# EUROPEAN PATENT APPLICATION

(11) **EP 2 022 460 A1**
(43) Date of publication of application: **11.02.2009**
(21) Application number: 08250036.4
(22) Date of filing: 04.01.2008
(51) Int. Cl.: A61H 23/02

(54) **Heatable massage head**

(30) Priority: 09.08.2007 CN 200720055306 U; 07.09.2007 CN 200720056694 U
(71) Applicant: Yuan, Shifeng, Buhe Town, Gongan County Hubei 434300 (CN)
(72) Inventor: Yuan, Shifeng, Buhe Town, Gongan County Hubei 434300 (CN)
(74) Representative: Forrester, Simon Joseph

(57) **Abstract**

A heatable massage head includes a massage-head shell, a heating means, a heating-means fixing seat, a massage-head fixing seat, and a connection means. The connection means has one end connecting to the heating means and the other end passing through the heating-means fixing seat and a massage-head fixing seat to contact with ring electrodes of a massage device rotatably. Thus, the heating means can continuously connect with the massage device mechanically and electrically while rotating. Therefore, the present heatable massage head not only can bring thermotherapy stimulation, but also can perform rotatably kneading and pressing massage on a relative larger region of the human body, thus achieving much better healthcare as well as therapeutic effects. In addition, the connection means comprises hollowed conductive metal rods to permit the conductive elastic elements to be inserted thereinto. Such connection is more reliable, and such connection means can lower cost.

## Description

The present invention relates generally to a massage head for a massage device, and more particularly to a heatable massage head.

### BACKGROUND OF THE INVENTION

With the improvement of human living standards, society rhythm has quickened and work pressure has increased, so people need to relax themselves to relieve work pressure as well as physical and mental exhaustion, thus accordingly, a variety of body healthcare apparatus is widely applied in modem family. The most common healthcare apparatus is the massage device at present. The massage device utilizes a massage head to bring mechanical stimulation on various parts of a human body, effectively easing muscle comfortlessness, tension or similar feeling.

The most popular type of massage head for massage devices is the heatable massage head now, which provides thermotherapy healthcare for human body via heating operation of a heating pipe formed therein and massage operation of raised points formed thereon. One such massage head is disclosed in Chinese Patent No. 96243060.9, issued on Mar. 18, 1998, the massage head has a vibrating mechanism and a heating apparatus to provide tremor massage and thermotherapy care for human body. However, such massage head has some limitations. For example, because the massage head can only change the massage place by hand, it can not attain to massage on a relative larger region of the human body automatically, thereby failing to make rotatably kneading and pressing massage. Therefore, the prior massage head is unable to well achieve basic massage-manipulation results and fails to realize imitated manual-massage effects.

Accordingly, massage manufacturers are trying to develop a heatable and rotatable massage head to overcome the defects mentioned above to cater to the needs of the market. However, there is still no effective solution on how to establish mechanical and electrical connection between the heating apparatus of the massage head and the massage device while rotating.

### SUMMARY OF THE INVENTION

It is an object of the present invention to seek to provide a heatable massage head.

It is envisaged that, in use, the heatable massage head is rotatably connected with a massage device and a heating means of the massage head could keep continuously electrical connection with the massage device while rotating, thus enabling the massage device not only to perform rotatably kneading and pressing massage, but also to conduct thermotherapy care, thus achieving much better healthcare as well as therapeutic effects.

According to the present invention there is provided a heatable massage head. The heatable massage head is adapted for mechanical and electrical connection with a massage device. The massage device has a rotating shaft and two ring electrodes around the rotating shaft. The heatable massage head comprises a massage-head shell, a heating means, a heating-means fixing seat, a massage-head fixing seat and a connection means. The heating means has a circuit board and a heating element electrically connecting to the circuit board. The massage-head shell and the circuit board are both fixed to the heating-means fixing seat. The massage-head shell encloses the heating means. The massage-head fixing seat is rotatably attached to the rotating shaft. The heating-means fixing seat is fixed to the massage-head fixing seat. One end of the connection means connects to the circuit board, the other end of the connection means passes through the heating-means fixing seat and the massage-head fixing seat to rotatably contact with the ring electrodes.

In an embodiment of the present invention, the connection means comprises a pair of electric conduction rods and a pair of guide sleeves slidably accommodating the corresponding electric conduction rods. The pair of electric conduction rods each has one end thereof electrically connected to the circuit board and the other end thereof rotatably contacted with the corresponding ring electrode. The guide sleeves are fixed to the massage-head fixing seat.

Preferably, the electric conduction rods are carbon rods or conductive metal rods. Also preferably, the conductive metal rods are copper rods, iron rods or aluminum rods.

In another embodiment of the present invention, the connection means further comprises a pair of conductive elastic elements. The pair of conductive elastic elements each has one end thereof electrically connected to the corresponding electric conduction rod and the other end thereof electrically connected to the circuit board.

Preferably, the conductive elastic element is a spring.

In another embodiment of the present invention, the conductive metal rods are hollow. Each conductive elastic element has one end thereof connected to the circuit board and the other end thereof inserted into the corresponding hollowed conductive metal rod to establish mechanical and electrical connection with the hollowed conductive metal rod.

Preferably, the conductive metal rods are copper rods, iron rods or aluminum rods.

In another embodiment of the present invention, each hollowed conductive metal rod has a cylindrical accommodating groove. The accommodating groove has one end thereof open for permitting the corresponding elastic element to be inserted into the accommodating groove therefrom and the other end thereof closed to withstand the corresponding elastic element.

In still another embodiment of the present invention, the connection means further comprises a pair of conductive pads. The pair of conductive pads both are formed on the massage-head fixing seat and connect to the circuit board electrically, the pair of conductive elastic elements connect with and stand against the corresponding conductive pads.

In yet another embodiment of the present invention, the connection means further comprises a pair of leads. The pair of leads each has one end thereof connected to the circuit board and the other end thereof passed through the heating-means fixing seat and the massage-head fixing seat to connect with the corresponding conductive pad.

Preferably, the heating element is a positive temperature coefficient heating plate or an incandescent lamp.

In another embodiment of the present invention, the heating means further comprises a heat transfer plate. The heat transfer plate is disposed between the heating element and an inner wall of the massage-head shell.

Preferably, the heatable massage head further comprises a plurality of light-emitting diode lamps mounted on and electrically connected to the circuit board for providing lighting effects for users.

The present invention employs a connection means which is able to rotatably contact with the external ring electrodes of a massage device, thus ensuring the heatable massage head to rotatably connect with the massage device during operation, and thereby enabling the heating means of the massage head to continuously connect with the massage device mechanically and electrically while the massage head rotating, which accordingly, assists to bring thermotherapy stimulation on the human muscles. Besides, the automatically rotatable massage head could perform rotatably kneading and pressing massage on a relative larger region of the human body, thereby not only able to achieve basic massage-manipulation results, but also able to imitate manual massage, and thus achieving better healthcare and therapeutic effects.

In addition, the connection means of the present invention could comprise hollowed conductive metal rods and conductive elastic elements respectively inserted into the corresponding hollowed conductive metal rods, thus the connection between the elastic element and the corresponding hollowed conductive metal rods might be much reliable. In such case, the massage device equipped with such massage head possesses high quality but low expenses. As for corporation, the massage head of the present invention lowers manufacturing cost but enhances competitive competence. As for users, the massage head of the present invention well meets people's needs for varieties of massage ways.

Other aspects, features, and advantages of this invention may become apparent from the following detailed description when taken in conjunction with the accompanying drawings, which are a part of this disclosure and which illustrate, by way of example, principles of this invention.

### BRIEF DESCRIPTION OF THE DRAWINGS

The accompanying drawings facilitate an understanding of the described embodiments of this invention. In such drawings:

FIG. 1 is a perspective view of a heatable massage head according to a first embodiment of the present invention, also showing a massage device matching with the heatable massage head;

FIG. 2 is an exploded, perspective view of the heatable massage head of FIG. 1;

FIG. 3 is an exploded, perspective view of the heatable massage head of FIG. 1 seen from another angle;

FIG. 4 is a perspective view of a heatable massage head according to a second embodiment of the present invention, also showing a massage device matching with the heatable massage head;

FIG. 5 is an exploded, perspective view of the heatable massage head of FIG. 4;

FIG. 6 is an exploded, perspective view of the heatable massage head of FIG. 4 seen from another angle;

FIG. 7 is a perspective view of a hollowed conductive metal rod of the heatable massage head shown in FIG. 5; and

FIG. 8 is a sectional view of a conductive pad, a spring and the hollowed conductive metal rod of the heatable massage head shown in FIG. 5 after assembled.

### DETAILED DESCRIPTION OF ILLUSTRATED EMBODIMENTS

Various preferred embodiments of the invention will now be described, by way of example, with reference to the figures, wherein like reference numerals designate similar parts throughout the various views.

In general terms, there is provided a heatable massage head. The heatable massage head employs a connection means which is able to rotatably contact with external ring electrodes of a massage device, for ensuring a heating means of the massage head continuously connects with the massage device mechanically and electrically while rotating. Therefore, the present heatable massage head could not only bring thermotherapy stimulation on human muscles, but also could perform rotatably kneading and pressing massage on a relative larger region of the human body and achieve better healthcare and therapeutic effects due to that the massage head could rotate automatically.

The heatable massage head generally comprises a massage-head shell, a heating means, a heating-means fixing seat, a massage-head fixing seat and a connection means. The connection means might comprise a pair of electric conduction rods and a pair of guide sleeves slidably accommodating the corresponding electric conduction rods. The electric conduction rods could be carbon rods or conductive metal rods and the conductive metal rods are preferably copper rods, iron rods or aluminum rods. Specifically, the conductive metal rod may be hollow.

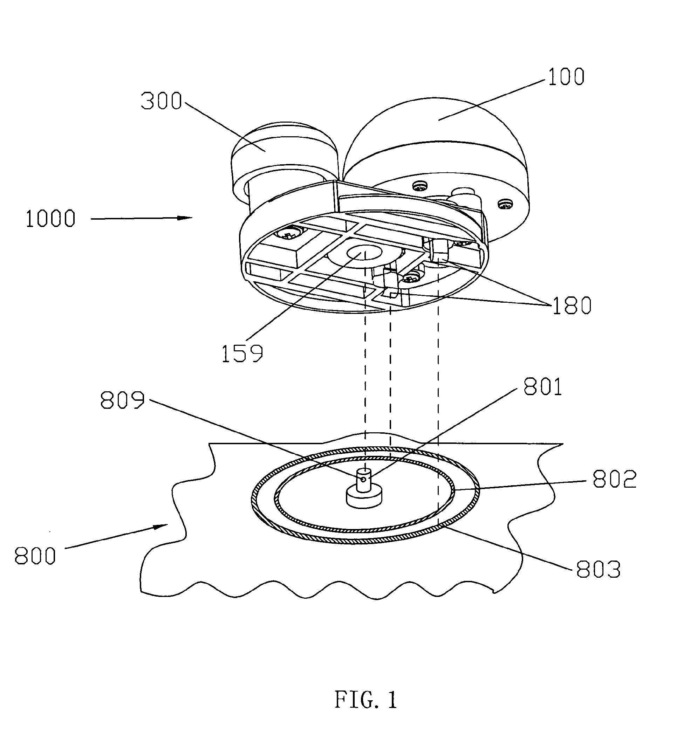

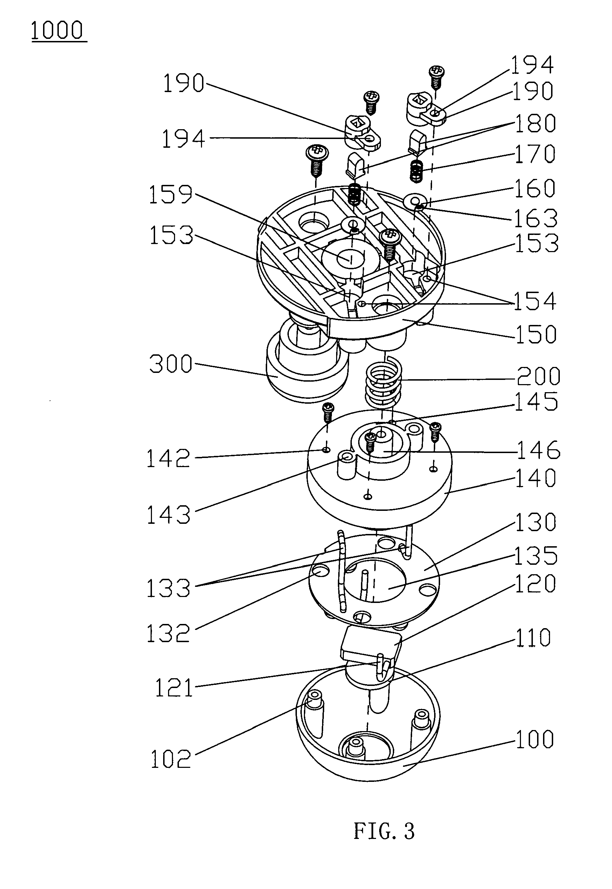

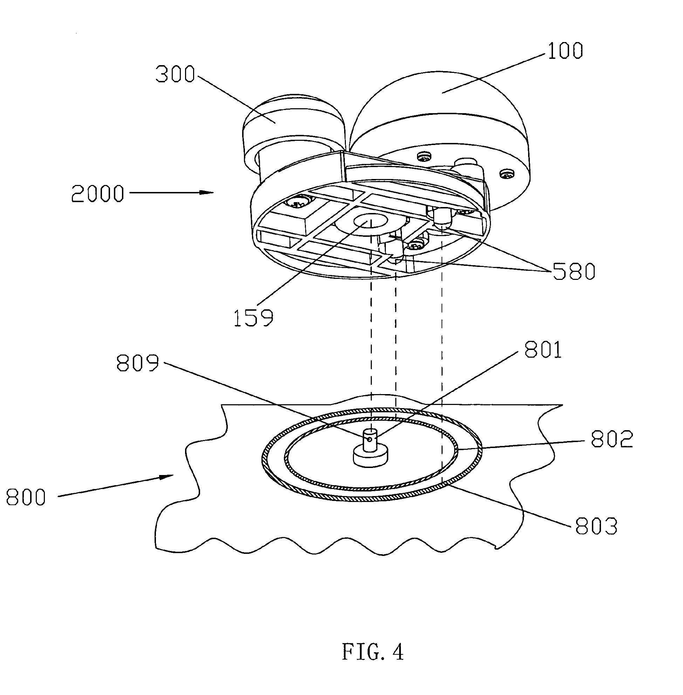

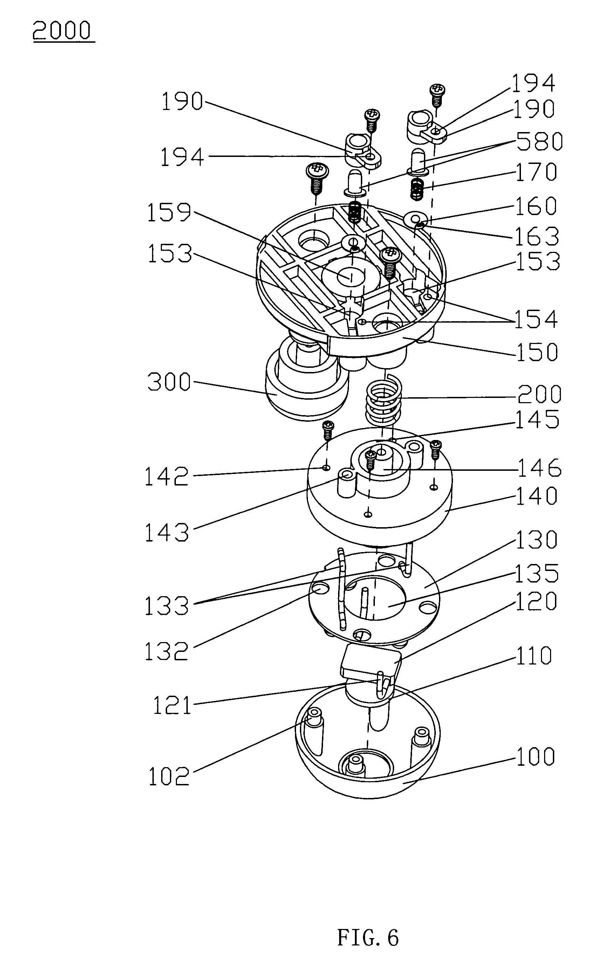

More specifically, FIGS. 1-3 show a heatable massage head 1000 according to a first embodiment of the present invention, wherein electric conduction rods of the heatable massage head 1000 are carbon rods. FIGS. 4-8 show a heatable massage head 2000 according to a second embodiment of the present invention, wherein the electric conduction rods of the heatable massage head 2000 are hollowed conductive metal rods.

Referring firstly to FIG. 1, the heatable massage head 1000 has a specially designed massage-head shell 100 capable of rotating while operating thermotherapy care and performing kneading and pressing massage and an additional massage-head shell 300 capable of rotation. The heatable massage head 1000 is adapted for establishing mechanical and electrical connection with a massage device 800. The massage device 800 includes a rotating shaft 801 and a pair of ring electrodes 802, 803 around the rotating shaft 801. The heatable massage head 1000 includes a pair of carbon rods 180 and a mounting hole 159. The rotating shaft 801 of the massage device 800 provides a mounting hole 809 at a position thereof corresponding to the mounting hole 159 of the massage head 1000. The heatable massage head 1000 and the massage device 800 can be assembled together rotatably by a common connector, such as a bolt passing through the mounting hole 159 of the massage head 1000 and the mounting hole 809 of the rotating shaft 801. After assembled, the two carbon rods 180 of the heatable massage head 1000 could electrically connect with and stand against the ring electrodes 802, 803 of the massage device 800.

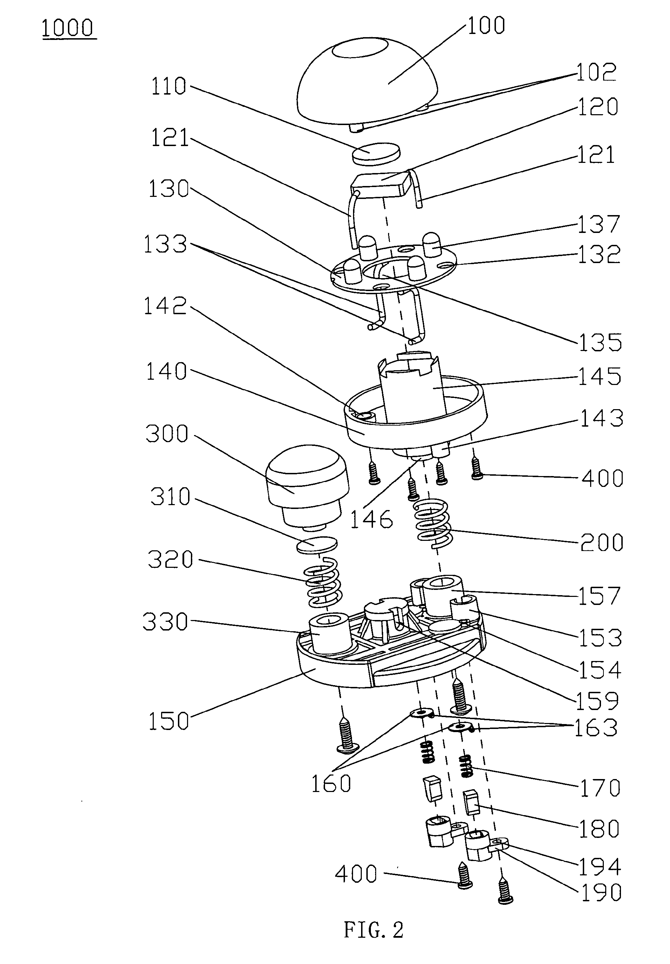

Referring to FIGS. 2-3, the heatable massage head 100 includes the massage-head shell 100, the additional massage-head shell 300, a heating means, a plurality of light-emitting diode lamps 137, a heating-means fixing seat 140, a massage-head fixing seat 150 and a connection means. The heating means includes a circuit board 130 and a heating element electrically connected to the circuit board 130. The heating element includes a positive temperature coefficient (PTC) heating plate 120 and a heat transfer plate 110. The heat transfer plate 110 is disposed on the positive temperature coefficient heating plate 120. The light-emitting lamps 137 are mounted on and electrically connected to the circuit board 130 for lighting effects. The massage-head shell 100 and the circuit board 130 are fixed to the heating-means fixing seat 140. The massage-head shell 100 encloses the heating means. The massage-head fixing seat 150 is rotatably attached to the rotating shaft 801 as mentioned above. The heating-means fixing seat 140 and the additional massage-head shell 300 are fixed on the massage-head fixing seat 150. The connection means has one end thereof connecting to the circuit board 130 and the other end thereof passing through the heating-means fixing seat 140 and the massage-head fixing seat 150 to rotatably connect with the ring electrode 802/803.

The connection means includes the pair of carbon rods 180, a pair of guide sleeves 190 slidably accommodating the corresponding carbon rods 180, a pair of springs 170, a pair of conductive pads 160 and a pair of leads 133. The pair of lead 133 each has one end thereof electrically connecting to the circuit board 130 and the other end thereof passing through the heating-means fixing seat 140 and the massage-head fixing seat 150 to connect with the corresponding conductive pad 160. The spring 170 has one end thereof connecting with and standing against the corresponding conductive pad 160 and the other end thereof electrically connecting to the corresponding carbon rod 180. In such case, the spring 170 could be made integratedly with the conductive pad 160. One end of the carbon rod 180 opposite the end thereof connected to the spring 170 rotatably contacts with the ring electrodes 802, 803. The carbon rod 180 is fixed to the massage-head fixing seat 150. Understandably, the spring 170 of the connection means of the subject embodiment may have some alternative elastic elements so long as the carbon rod 180 could be electrically connected to the corresponding conductive pad 160 bufferingly.

The massage-head shell 100 provides four fixing holes 102. The positive temperature coefficient heating plate 120 provides two pins 121. The circuit board 130 has a circuit-board hole 135, four fixing holes 132 and four light-emitting diode lamps 137. The heating-means fixing seat 140 provides a first sleeve 145, a second sleeve 146 formed in the first sleeve 145, two lead holes 143 and four fixing holes 142. The circuit-board holes 135 of the circuit board 130 of the heating means matches with the first guide sleeve 145 of the heating-means fixing seat 140. The fixing holes 102 of the massage-head shell 100, the fixing holes 132 of the circuit board 130 of the heating means, and the fixing holes 142 of the heating-means fixing seat 140 match with each other in order to be assembled together. The massage-head fixing seat 150 includes a sleeve 157 sandwiched between the first sleeve 145 and the second sleeve 146 of the heating-means fixing seat 140, two lead holes 153, two fixing holes 154, a mounting hole 159 and an additional hole 330. The mounting hole 159 of the massage-head fixing seat 140 is used for rotatably mounting the massage-head fixing seat 150 onto the massage device 800. The two conductive pads 160 of the connection means each form a contact 163. The two guide sleeves 190 each form a hole 194. The fixing hole 154 of the massage-head fixing seat 150 matches with the hole 194 of the guide sleeve 190. The connection further comprises two leads 133. In the subject embodiment, additional spring 320 and additional spring pad 310 is used for mounting the additional massage-head shell 300 onto the massage-head fixing seat 150.

The following details illustrate assembly of the heatable massage head 1000:

First, assembly of the massage-head shell 100, the heating means and the heating-means fixing seat 140 will be illustrated. The top end of the first sleeve 145 of the heating-means fixing seat 140 is passed through the circuit-board hole 135 of the circuit board 130 while the four fixing holes 132 of the circuit board 130 are respectively aligned with the four fixing holes 142 of the heating-means fixing seat 140. At the same time, the two leads 133 of the connection means each has one end thereof electrically connecting to the circuit board 130 and the other end thereof passing through the lead hole 143 of the heating-means fixing seat 140. The two pins 121 of the positive temperature coefficient heating plate 120 are electrically connected to the circuit board 130. The positive temperature coefficient heating plate 120 is fixed on a top end of the first sleeve 145. The heat transfer plate 110 is formed on the positive temperature coefficient heating plate 120. The massage-head shell 100 encloses the heat transfer plate 110, the positive coefficient heating plate 120 and the circuit board 130 of the heating means. At the same time, the four fixing holes 102 of the massage-head shell 100 are aligned with the four fixing holes 132 of the circuit board 130. In such fashion, the massage-head shell 100, the circuit board 130 and the heating-means fixing seat 140 can be assembled together by the four screws 400 sequentially passing through the corresponding fixing holes 142 of the heating-means fixing seat 140, the fixing holes 132 of the circuit board 130 and the fixing holes 102 of the massage-head shell 100.

Assembly of the massage-head fixing seat 150, the heating-means fixing seat 140 and the additional massage-head shell 300 is carried out as follows:

The two leads 133 of the connection means respectively pass through the lead holes 153 of the massage-head fixing seat 150. The spring 200 makes one end thereof accommodated by the sleeve 157 of the massage-head fixing seat 150 and the other end thereof stand against the second sleeve 146 of the heating-means fixing seat 140. The sleeve 157 of the massage-head fixing seat 150 is inserted between the first sleeve 145 and the second sleeve 146 of the heating-means fixing seat 140. The massage-head fixing seat 150 is mechanically connected with the heating-means fixing seat 140 at the sleeve 157 thereof by screws 400. The additional spring 320 is inserted into the additional sleeve 330 of the massage-head fixing seat 150. The additional spring pad 310 is formed on the additional spring 320 and the additional massage-head shell 300 is fixed to the additional spring pad 310. The additional massage-head shell 300 is assembled with the massage-head fixing seat 150 by the screws 400 passing through the additional sleeve 330.

Finally, assembly of the connection means and the massage-head fixing seat 150 will be illustrated. The two leads 133 of the connection means are electrically connected to the corresponding contacts 163 of the conductive pads 160 via traditional means. The spring 170 makes one end thereof connect with and stand against the conductive pad 160 and the other end thereof electrically connect with the carbon rods 180. The carbon rod 180 is partially inserted into the guide sleeve 190, making the carbon rod 180 partially exposed outside. Therefore, the guide sleeve 190 could slidably accommodate the carbon rod 180. The two screws 400 sequentially pass through the corresponding holes 194 of the guide sleeve 190 and the fixing holes 154 of the massage-head fixing seat 150 to fix the guide sleeve 190 on the massage-head fixing seat 150.

In operation, the massage-head fixing seat 150 keeps rotating around the rotating shaft 801 of the massage device 800, thus the carbon rods 180 of the connection means rotatably could contact with the ring electrodes 802, 803 of the massage device 800 automatically. The positive temperature coefficient heating plate 120 and the light-emitting diode lamps 137 in rotation could establish continuously electrical and mechanical connection with the massage device 800. When electric current sequentially passes the carbon rods 180, the springs 170, the conductive pads 160, the leads 133 and the circuit board 130 of the heating means, the positive temperature coefficient heating plate 120 begins to heat up and the light-emitting diode lamps 137 begins to produce lighting effects, and then the heat transfer plate 110 on the positive temperature coefficient heating plate 120 starts to transfer heat continuously, thus the massage head 1000 in rotation could stimulate the strained muscles by heat to realize thermotherapy care and allow the user to enjoy lighting effects. Hence, the heatable massage head 1000 enables massage places of human body to keep on changing, achieving rotatably kneading and pressing massage on a relative larger region of the human body and getting better healthcare and therapeutic effects. In addition, the heatable massage head of the subject embodiment provides the additional massage-head shell 300 capable of rotation, thus effects of rotatable massage are further enhanced. It will be appreciated that, alternatively, the positive temperature coefficient heating plate 120 can be replaced with incandescent lamps that have the same function of producing heat.

Thereafter, the heatable massage head 2000 of the second embodiment of the present invention will be illustrated.

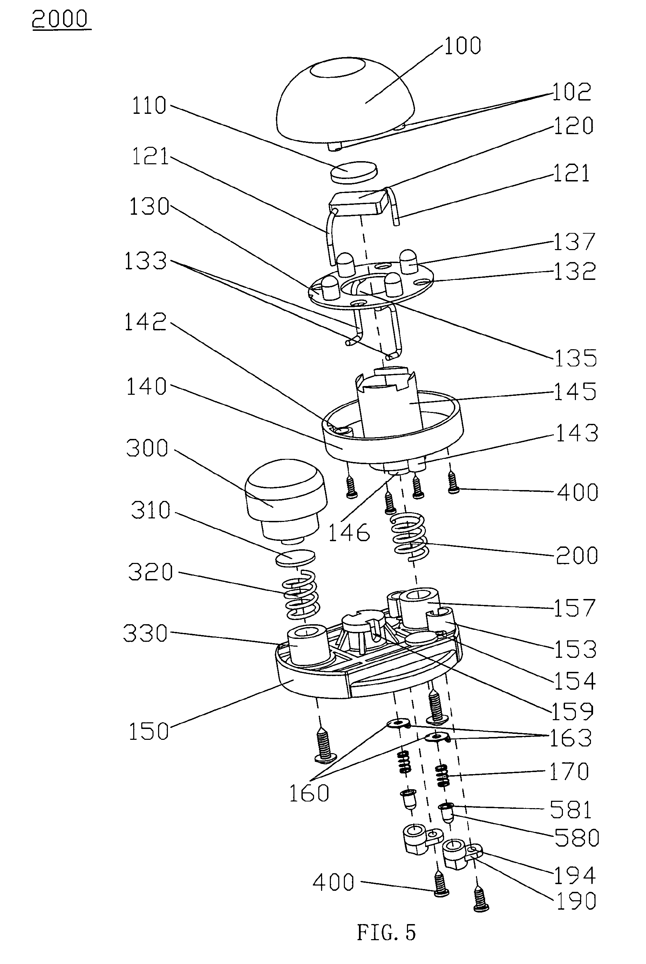

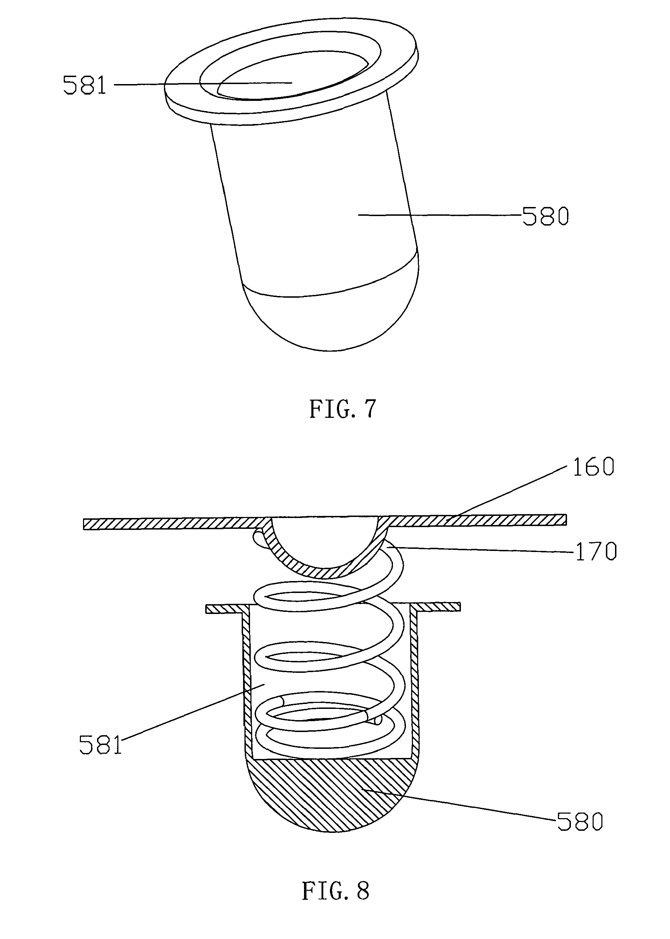

Referring to FIG. 4, the heatable massage head 2000 is also adapted for establishing mechanical and electrical connection with the massage device 800, the feature of which is explained mentioned above. The heatable massage head 2000 includes a massage-head shell 100, a heating means, a plurality of light-emitting diode lamps 137, a heating-means fixing seat 140, a massage-head fixing seat 150 and a connection means. Referring to FIGS. 5-6, the heatable massage head 2000 in the subject embodiment is similar to the heatable massage head 1000 of the first embodiment mentioned above except that the connection means of the heatable massage head 2000 employs a pair of hollowed conductive metal rods 580 as electric conduction rods while the connection means of the heatable massage head 1000 employs a pair of carbon rods 180 as electric conduction rods. The hollowed conductive metal rods 580 are slidably accommodated by guide sleeves 194. In the embodiment, referring to FIG. 7, the hollowed conductive metal rods 580 each has a cylindrical accommodating groove 581. The accommodating groove 581 has one end thereof open for permitting the corresponding spring 170 to be inserted into the accommodating groove 581 therefrom and the other end thereof closed to withstand the spring 170. Referring to FIG. 8, the spring 170 has one end thereof connecting with and standing against a conductive pad 160 and the other end thereof inserted into the accommodating groove 581 of the hollowed conductive metal rod 580.

Assembly of additional massage-head shell 300, the massage-head shell 100, the heating means and the heating-means fixing seat 140, and the massage-head fixing seat 150 is implemented as the same way as those of the heatable massage head 1000 in the first embodiment, which are omitted. It is noted that each of two leads 133 has one end thereof electrically connecting to the circuit board 130 of the heating means and the other end thereof sequentially passing through the lead hole 143 of the heating-means fixing seat 140, the lead holes 153 of the massage-head fixing seat 150 during the above-mentioned assembly process of the heatable massage head 2000. The following will specifically explain assembly of the connection means relative to the other components of the heatable massage head 2000. The two leads 133 then each makes the other end thereof electrically connect to the corresponding contact 163 of the conductive pad 160. The spring 170 makes one end thereof connect with and stand against a conductive pad 160 and the other end thereof inserted into the accommodating groove 581 of the hollowed conductive metal rods 580. The hollowed conductive metal rods 580 are partially inserted into the guide sleeve 190, making the hollowed conductive metal rods 580 partially exposed outside. Therefore, the guide sleeve 190 could slidably accommodate the conductive metal rod 180. Two screws 400 sequentially pass through the corresponding holes 194 of the guide sleeve 190 and the fixing holes 154 of the massage-head fixing seat 150 to fix the guide sleeve 190 on the massage-head fixing seat 150.

In operation, the heatable massage head 2000 keeps rotating around the rotating shaft 801 of the massage device 800, thus the hollowed conductive metal rods 580 of the connection means rotatably could contact with the ring electrodes 802, 803 of the massage device 800 automatically. Accordingly, the positive temperature coefficient heating plate 120 and the light-emitting diode lamps 137 in rotation could establish continuously electrical and mechanical connection with the massage device 800. When electric current sequentially passes the hollowed conductive metal rods 580, the springs 170, the conductive pads 160, the leads 133 and the circuit board 130 of the heating means, the positive temperature coefficient heating plate 120 begins to heat up and the light-emitting diode lamps 137 begins to produce lighting effects, and then the heat transfer plate 110 on the positive temperature coefficient heating plate 120 starts to transfer heat continuously, thus the massage head 2000 in rotation could stimulate the strained muscles by heat to realize thermotherapy care and allow the user to enjoy lighting effects. Hence, the heatable massage head 2000 achieves rotatably kneading and pressing massage on a relative larger region of the human body and getting better healthcare and therapeutic effects. In addition, as the spring 170 is inserted into the accommodating groove 581 of the hollowed conductive metal rod 580 of the connection means, the motion region of the spring 170 is confined to a limited space defined by the accommodating groove 581, thus the connection between the springs 170 and the corresponding hollowed conductive metal rods 580 are much reliable. In this case, the massage device 800 equipped with the heatable massage head 2000 possesses high quality but low expenses. Thus, it is envisaged that the massage head 2000 will lower manufacturing cost and enhance competitive competence. In addition, it is envisaged that the massage head 2000 will meet users' needs for a variety of massage ways.

The foregoing description of the present invention has been presented for purposes of illustration and description. It is not intended to be exhaustive or limit the invention to the precise form disclosed, and many modifications and variations are possible in light of the above teaching. Such modifications and variations that may be apparent to those skilled in the art are intended to be included within the scope of this invention as defined by the accompanying claims.

## Claims

1. A heatable massage head [1000, 2000] configured for mechanical and electrical connection with a massage device [800], the massage device having a rotating shaft [801] and two ring electrodes [802, 803] around the rotating shaft [801], the heatable massage head [1000, 2000] comprising:
a massage-head shell [100];
a heating means having a circuit board [130] and a heating element electrically connecting to the circuit board [130];
a heating-means fixing seat [140], the massage-head shell [100] and the circuit board [130] being both fixed to the heating-means fixing seat [140], the massage-head shell [100] enclosing the heating means;
a massage-head fixing seat [150] rotatably attached to the rotating shaft [801], the heating-means fixing seat [140] being fixed to the massage-head fixing seat [150]; and
a connection means, one end of the connection means connecting to the circuit board [130], the other end of the connection means passing through the heating-means fixing seat [140] and the massage-head fixing seat to rotatably contact with the ring electrodes [802, 803].

2. A heatable massage head [1000, 2000] according to claim 1, wherein the connection means comprises a pair of electric conduction rods [180, 580] and a pair of guide sleeves [190, 194] slidably accommodating the electric conduction rods [180, 580], the pair of electric conduction rods [180, 580] each has one end thereof electrically connected to the circuit board [130] and the other end thereof rotatably contacted with the corresponding ring electrode [802, 803], the guide sleeves [190, 194] are fixed to the massage-head fixing seat [150].

3. A heatable massage head [1000, 2000] according to claim 2, wherein the electric conduction rods are carbon rods [180] or conductive metal rods [580].

4. A heatable massage head [2000] according to claim 3, wherein the conductive metal rods [580] are copper rods, iron rods, or aluminum rods.

5. A heatable massage head [1000, 2000] according to claim 3, wherein the connection means further comprises a pair of conductive elastic elements [170], the pair of conductive elastic elements [170] each has one end thereof electrically connected to the corresponding electric conduction rod [180, 580] and the other end thereof electrically connected to the circuit board [130].

6. A heatable massage head [1000, 2000] according to claim 5, wherein the conductive elastic element is a spring [170].

7. A heatable massage head [2000] according to claim 5, wherein the conductive metal rods [580] are hollow, and each conductive elastic element [170] has one end thereof connected to the circuit board [130] and the other end thereof inserted into the corresponding hollowed conductive metal rod [580] to establish mechanical and electrical connection with the hollowed conductive metal rod [580].

8. A heatable massage head [2000] according to claim 7, wherein each hollowed conductive metal rod [5 80] has a cylindrical accommodating groove [581], and the accommodating groove [581] has one end thereof open for permitting the corresponding elastic element [170] to be inserted into the accommodating groove [581] therefrom and the other end thereof closed to withstand the corresponding elastic element [170].

9. A heatable massage head [1000, 2000] according to claim 5, wherein the connection means further comprises a pair of conductive pads [160], and the pair of conductive pads [160] both are formed on the massage-head fixing seat [140] and connect to the circuit board [130] electrically, the pair of conductive elastic elements [170] connect with and stand against the corresponding conductive pads [160].

10. A heatable massage head [1000, 2000] according to claim 9, wherein the connection means further comprises a pair of leads [133], the pair of leads [133] each has one end thereof connected to the circuit board [130] and the other end thereof passed through the heating-means fixing seat [140] and the massage-head fixing seat [150] to connect with the corresponding conductive pad [160].

11. A heatable massage head [1000, 2000] according to claim 1, wherein the heating element comprises a positive temperature coefficient heating plate [120] or an incandescent lamp.

12. A heatable massage head [1000, 2000] according to claim 1, wherein the heating means further comprises a heat transfer plate [110], and the heat transfer plate [110] is disposed between the heating element [120] and an inner wall of the massage-head shell [100].

13. A heatable massage head [1000, 2000] according to claim 1, further comprising a plurality of light-emitting diode lamps [137] mounted on and electrically connected to the circuit board [130].
